# EUROPÄISCHE PATENTSCHRIFT

(11) **EP 0 774 245 B1**
(45) Veröffentlichungstag und Bekanntmachung des Hinweises auf die Patenterteilung: **02.04.2003**
(21) Anmeldenummer: 96117713.6
(22) Anmeldetag: 06.11.1996
(51) Int. Cl.: A61H 3/06, A61F 9/08

(54) **Orientierungshilfe für Sehbehinderte**
Orientation aid for the visually impaired
Aide d'orientation pour mal-voyants

(30) Priorität: 16.11.1995 DE 19542678
(43) Veröffentlichungstag der Anmeldung: 21.05.1997
(73) Patentinhaber: Schrader, Jens, Dipl.-Ing., 70190 Stuttgart (DE)
(72) Erfinder: Schrader, Jens, Dipl.-Ing., 70190 Stuttgart (DE)
(74) Vertreter: Weiss, Peter, Dr. rer. nat.

(56) Entgegenhaltungen:
- DE-U- 9 415 210
- GB-A- 2 287 535
- US-A- 5 144 294
- US-A- 5 307 137
- US-A- 5 406 491
- PATENT ABSTRACTS OF JAPAN vol. 015, no. 128 (C-0818), 28.März 1991 & JP 03 012156 A (MURATA MFG CO LTD), 21.Januar 1991,

## Beschreibung

Die vorliegende Erfindung betrifft eine Orientierungshilfe für Sehbehinderte und blinde Menschen.

Derartige Orientierungshilfen sind in vielfältiger Form und Ausführung auf dem Markt bekannt und gebräuchlich. Beispielsweise ist ein einfacher Stab bekannt, mit welchem ein Blinder einen Weg, eine Hauswand, eine Straße, Treppe od.dgl. Hindernisse abtastet. Nachteilig an solchen Stäben ist, daß diese im unteren Bereich zwischen Hüfte und Fuß zwar ein Ertasten von Hindernissen zulassen, jedoch im Oberkörper und Kopfbereich eine gewisse Unsicherheit vor Hindernissen gegeben ist und der Blinde nur auf seine Sinnesorgane und Reflexe angewiesen ist. Daher besteht für den blinden Menschen eine große Gefahr, beim Bewegen sich an Hindernissen in dieser Höhe zu verletzen.

Ferner sind Blinde beim Gehen darauf angewiesen, eine Führung entlang Gehsteigen, Straßen, Wänden und dgl. mittels einem Stab abzutasten, sodaß nur so ein Weg in eine bestimmte Richtung ermittelt werden kann. Jedoch sind nicht immer Gehsteige, Wände bzw. Häuserwände als Abtast- und Führungshilfen vorhanden. Bspw. ist ein Überqueren einer Straße für Blinde sehr schwierig, da die Richtung zur anderen Straßenseite hin exakt ohne jegliche Führung eingehalten werden muß. Auch ein Geradeausgehen ist ohne Führung nicht möglich.

Die US 5,406,491 beschreibt eine Vorrichtung zum Erkennen einer Wanderroute, wobei ein Anfangspunkt und ein Zielpunkt eingegeben sind. Entsprechende Abweichungen von der Reiseroute lassen sich nach Standortermittlung über GPS zum Erreichen eines bestimmten Zieles korrigieren.

Der vorliegenden Erfindung liegt die Aufgabe zu Grunde, eine Orientierungshilfe zu schaffen, welche die o.g. Nachteile beseitigt, und bei welcher eine gerade Gehrichtung eingehalten werden kann, und welche einen Oberkörperbereich unmittelbar vor dem Körper vor Hindernissen schützt.

Zur Lösung dieser Aufgabe führt die Merkmale des Anspruches 1.

Diese Erfindung gestattet, daß ein Kompaß, welcher bevorzugt im hinteren Teil eines Gürtels, insbesondere in der Nähe des Rückgrades angeordnet ist, eine bestimmte Gehrichtung erkennt. Damit dieses Erkennen in elektrische Signale umsetzbar ist, ist dieser bevorzugt als elektrischer Kompaß ausgebildet. Ferner sind dem Gürtel der Orientierungshilfe zumindest zwei Signaleinrichtungen zugeordnet, die bevorzugt rechts und links der Gürtelschnalle angeordnet sind. Wird der Kompaß in eine gewünschte Gehrichtung ausgerichtet, so wird eine Koordinate nach dem Ausrichten in einem Prozessor, welcher ebenfalls dem Gürtel zugeordnet ist, wenn gewünscht, abgespeichert. Der Prozessor vergleicht ständig während dem anschließenden Gehen den aktuellen Kompaßwert mit dem gespeicherten und gibt je nach Änderung ein Signal an die eine oder andere Signaleinrichtung ab. Ist die aktuelle Koordinatenabweichung bzw. Richtungsabweichung beim Gehen größer als der abgespeicherte Wert, so wird je nach Anordnung der Signaleinrichtungen eine der beiden betätigt, so daß der Blinde genau weiß, in welche Richtung er gehen muß, um die fest eingespeicherte und gewünschte Richtung wieder zu erreichen. Dies ist insbesondere beim Überqueren von Straßen, Wegen, Wiesen od. dgl. vorteilhaft, da der Blinde dort keine Möglichkeit der Orientierung beispielsweise durch Gehsteige, Wände od. dgl. hat.

Die Signaleinrichtungen, welche von einem bevorzugt wiederaufladbaren Akku betrieben werden, können mittels Vibratoren Signale an den Körper übermitteln, wobei jedoch auch an akkustische Signale über Lautsprecher oder elektrische Signale über Stromreize direkt an die Haut gedacht ist.

Der Gürtel ist bevorzugt aus elastischem Material, wobei alle Bauteile, wie Akku, Kompaß, Signaleinrichtungen elektrisch innerhalb des Gürtels miteinander verbunden sind und unter einem Pullover ggfs. auch auf der Haut getragen werden können.

In einem weiteren Ausführungsbeispiel der vorliegenden Erfindung ist daran gedacht eine Orientierungshilfe zu schaffen, welche mittels Streifen und Seitenstreifen um den Halsbereich auf der Schulter aufliegt. Dabei übergreifen die Seitenstreifen die Schulter, wobei im hinteren Bereich der Streifen im Nackenbereich an der Schulter anliegt. Dort sind auch Kompaß, Prozessor und Akku und dgl. Einrichtungen untergebracht. Im vorderen Bereich sind auf den Seitenstreifen rechts und links Signalgeber angerodnet, die mit Schaltern versehen sind. Einer der beiden Schalter dient zum Ein- oder Ausschalten der Orientierungshilfe, wobei der andere Schalter optional die Art des Signales, welches dort angezeigt wird, entweder elektrisch, elektromechanisch oder akkustisch wählt.

Zusätzlich ist zumindest einem der Signalgeber ein Sensor zugeordnet, welcher einen vorderen Bereich vor dem Menschen überwacht. Der Sensor kann als Infrarot- und/oder Ultraschall- und/oder Mikrowellen- od. dgl.- Sensor ausgebildet sein. Der Sensor hat die Aufgabe, insbesondere den oberen Körperbereich, Schulter, Kopfbereich zu überwachen und dem blinden Menschen über ein akkustisches, elektrisches oder elektromechanisches Signal anzuzeigen, wenn in dem Bereich vor dem Oberkörper ein Hindernis auftritt.

Dieser Bereich bzw. die Reichweite dieses Sensors ist absichtlich sehr klein gewählt, wobei an Bereiche zwischen 0,5 bis 1.5 Meter vor dem Oberkörperbereich gedacht ist. Nur so hat der Blinde die Möglichkeit, nicht von Hindernissen in größerer Entfernung beeinflußt zu werden. Zusätzlich kann auch in diese Orientierungshilfe ein Kompaß eingebaut sein, welcher die oben beschriebenen Funktionen gleichfalls erfüllt.

Zur einfachen Bedienung der Orientierungshilfe ist an einem Stab ein Schalter vorgesehen, der die Aktivierung der Richtungsanzeige bzw. der Richtungskoordinate übernimmt und im Prozessor speichern kann. Die Übertragung an den Prozessor erfolgt über Funk, Infrarotstrahlung od. dgl..

Ferner soll im Rahmen der vorliegenden Erfindung liegen, daß in eine solche Orientierungshilfe ein GPS-System eingesetzt ist, welches nach einer bestimmten Auswahl von Koordinaten bzw. einem bestimmten Ziel dem Blinden über die Signalgeber die Richtung anzeigen kann. Gleichzeitig wird ein Geradeausgehen durch den Kompaß unterstützt.

Weitere Vorteile, Merkmale und Einzelheiten der Erfindung ergeben sich aus der nachfolgenden Beschreibung bevorzugter Ausführungsbeispiele sowie anhand der Zeichnung, diese zeigt in
Figur 1 eine perspektivische Ansicht einer erfindungsgemäßen Orientierungshilfe;
Figuren 2a und 2b perspektivisch dargestellte Ansichten von weiteren Ausführungsbeispielen der Orientierungshilfe nach Figur 1;
Figur 3 eine Ansicht der Orientierungshilfen gemäß den Figuren 1, 2a und 2b in Gebrauchslage;
Figur 4 einen erfindungsgemäßen Stab.

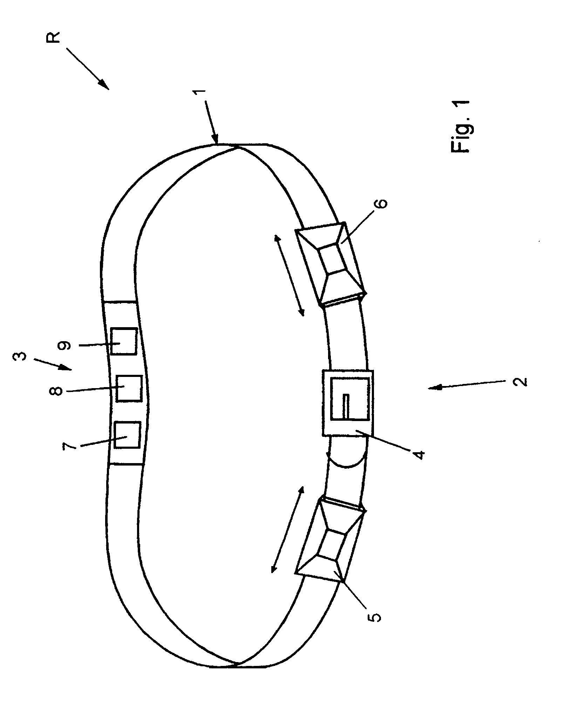

Gemäß Figur 1 weist eine erfindungsgemäße Orientierungshilfe R einen Gürtel 1 auf, welcher in einen vorderen Bereich 2 und hinteren Bereich 3 unterteilt ist. Der Gürtel 1 ist zum Anziehen um einen menschlichen Körper insbesondere im Hüftbereich geeignet. Dabei soll der vordere Bereich 2 am Bauch und der hintere Bereich 3 am Rücken eines menschlichen Körpers anliegen. Auch der umgekehrte Fall ist denkbar.

Damit der Gürtel 1 um einen menschlichen Körper gelegt werden kann, ist dieser vorher mittels eines Verschlusses 4 zu öffnen beziehungsweise wieder zu verschließen. Selbstverständlich liegt im Rahmen der Erfindung ein stufenloses Einstellen der Gürtelweite, sodaß dieser an unterschiedlich groß geformte Körper angepaßt werden kann.

Im vorderen Bereich 2 sind rechts und links des Verschlusses 4 gleichmäßig beabstandet Signaleinrichtungen 5 und 6 dem Gürtel 1 zugeordnet. Die Signaleinrichtungen 5, 6 stehen über eine hier nicht näher dargestellte elektrische Verbindung, welche in dem bevorzugt weich und gepolstert ausgebildeten Gürtel 1 in den hinteren Bereich 3 verläuft, mit einem Prozessor 7 in Verbindung. Der im hinteren Bereich 3 angeordnete Prozessor 7 erhält Signale von einem mittig daneben angeordneten elektrischen Kompaß 8. Ferner werden die Signaleinrichtungen 5, 6, der Prozessor 7 und der elektrische Kompaß 8 von einem im hinteren Bereich 3 angeordeneten bevorzugt wiederaufladbarem Akku 9 gespeist.

Die Funktionsweise der vorliegenden Erfindung ist folgende:

Für blinde Menschen ist es besonders schwierig eine bestimmte Richtung insbesondere beim Gehen beizubehalten, wenn sie beispielsweise keine richtungsweisenden Angaben oder Führungen wie Gehsteige, Häuserwände haben. Aber insbesondere beim Überqueren von Straßen oder Kreuzungen, wo weder ein Gehsteig noch eine Hauswand od.dgl. vorhanden ist, ist für den Blinden von großer Bedeutung, daß er quer direkt über die Straße geführt wird und diese im kürzesten Weg überquert.

Aber auch ein Geradeausgehen, beispielsweise entlang einer geraden Straße, kann mit dieser erfindungsgemäßen Orientierungshilfe leicht geschehen.

Der elektrische Kompaß 8, welcher bevorzugt mittig, also nahe des Rückgrades des menschlichen Körpers, im Gürtel 1 ausgerichtet ist, übermittelt, wenn gewünscht, auf ein Signal hin die aktuelle Koordinate im Kompaß 8 in Geh- bzw. Laufrichtung des menschlichen Körpers an den Prozessor 7.

Diese Koordinate wird auf ein bestimmtes Signal, welches von einem hier nicht näher gezeigten Schalter am Gürtel 1 oder an einer der Signaleinrichtungen 5, 6 ausgelöst wird, abgespeichert. Diese gespeicherte Koordinate wird dann als Referenzkoordinate zu den folgenden bzw. ändernden Koordinaten beim Gehen in eine andere Richtung als Meßgrundlage genommen.

Weicht nun von der gespeicherten Koordinate die fortlaufende Koordinate beim Gehen ab, die in dem elektrischen Kompaß 8 gemessen wird, so wird je nach Abweichung, ob darüber- oder darunterliegend ein Signal an die Signaleinrichtung 5 oder 6 übermittelt. Diese Signaleinrichtungen 5, 6 sollen die Signale in Form eines Vibrationsgeräusches, welches direkt auf der Haut beziehungsweise auf dem Körper spürbar ist, dem Blinden übermitteln. Weicht nun die Richtung von der ursprünglich abgespeicherten Richtung ab, ändert der Blinde dadurch seine Gehrichtung entweder nach rechts oder nach links, je nachdem welche Signaleinrichtung 5, 6 das entsprechende Signal übermittelt, bis die ursprünglich gespeicherte Richtung wieder erreicht ist.

Wird ein erneutes Signal wieder als Richtungsreferenz für eine neue Richtungsanzeige benötigt, so richtet sich der Blinde in die Richtung aus, in die er gehen möchte, und betätigt erneut den Prozessor 7, welcher die gewünschte Gehrichtung bzw. Richtungskoordinate des elektrischen Kompasses 8 als Referenz im Prozessor 7 speichert. Beim anschließenden Gehen werden Abweichungen, die von dem elektrischen Kompaß 8 an den Prozessor 7 übermittelt und erkannt werden, an den Signaleinrichtungen 5 und 6 in oben beschriebener Weise zur Anzeige gebracht, so daß der Blinde weiß, in welche Richtung er gehen muß, um die gewünschte Gehrichtung beizubehalten. Sind die Abweichungen zwischen gespeicherter Richtungskoordinate und tatsächlicher Richtungskoordinate beim Gehen sehr gering, so setzt die Signaleinrichtung 5, 6 nicht ein. Diese Toleranz soll hier einstellbar sein.

Im Rahmen der Erfindung soll jedoch auch liegen, daß die Signaleinrichtungen 5, 6 akustische Töne von sich geben können, die auch unterschiedlicher Tonlagen sein können, sodaß der Blinde direkt erkennt, er muß eine Richtung nach rechts oder nach links einschlagen.

Auch elektrische Signaleinrichtungen 5, 6 sind denkbar, die beispielsweise über elektrische Ströme ein Kribbeln auf der Haut des menschlichen Körpers erzeugen, sodaß der Blinde ebenfalls spürt, welche Richtung er einschlagen muß, um die gewünschte und vorher abgespeicherte Richtung wieder zu finden und einzuhalten. Vorteilig ist hier, daß dadurch keine Geräusche entstehen und der Blinde sich auf Geräusche seiner Umgebung konzentrieren kann.

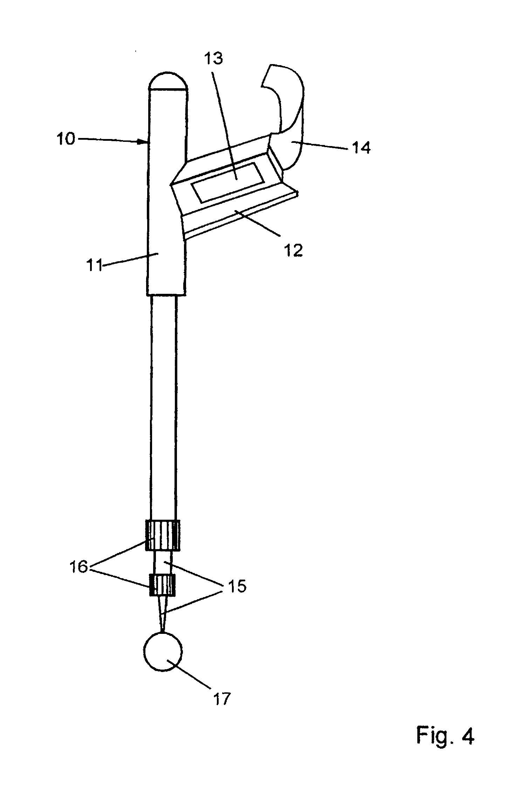

Auch ein Tastendruck eines Schalters 13 an einem Stab 10, wie in Figur 4 dargestellt, kann eine gewünschte Gehrichtung speichern. Der Stab 10 weißt einen Griff 11 auf, dem ein Führungselement 12 mit dem Schalter 13 zugeordnet ist. Das Führungselement 12 weißt ferner ein Band 14 auf, welches bspw. durch Einlage biegsamer Metalle od.dgl. an die Form eines Handgelenkes anpassbar ist. So liegt der Stab 10 leicht, sicher und geführt am Handgelenk in der Hand. Der Schalter 13 wird mit einem Finger einer Hand betätigt, sodaß in diesem Augenblick die Richtungskoordinate im Prozessor 7 gespeichert wird. Ein erneuter Druck auf diesen Schalter 13 schaltet die Orientierungshilfe R aus.

Damit der Blinde immer die aktuelle Schalterfunktion erkennt, werden sogenannte Hoch / Tiefschalter verwendet, deren Ein- oder Ausschaltzustand durch die Stellung des Schalters erkennbar ist. Auch andere Schalter, wie Kippschalter, sind hier denkbar.

Das Signal des Schalters 13 wird, wie hier nicht näher dargestellt, über Funk, Infrarotstrahlung, od.dgl. an den Gürtel 1 übermittelt. In diesem Fall wäre der Gürtel 1 mit einem entsprechenden Empfängerteil versehen.

Damit der Stab 10 für Menschen unterschiedlicher Größe variabel ausgebildet ist, weißt dieser teleskopartige Verlängerungen 15 auf, welche mittels Befestigungselementen 16 in jeder ausgefahrenen Lage gegenüber dem Griff 11 fixierbar sind. Am Ende des Stabes 10 ist als Stabspitze eine um die Stabachse leicht drehbare Kugel 17 angeordnet, die ein Pendeln erleichtert, ohne daß Tastfunktionen eingeschränkt werden.

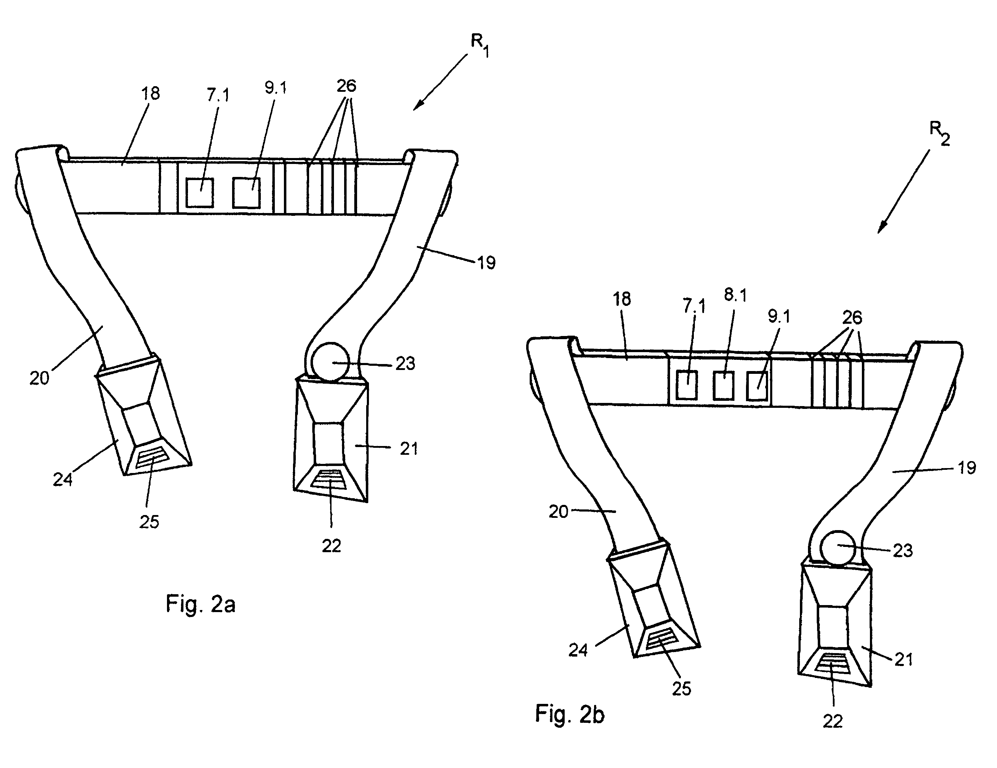

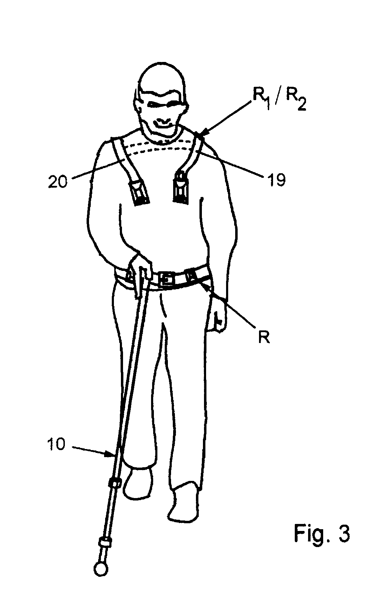

In einem weiteren Ausführungsbeispiel der vorliegenden Erfindung gemäß Figur 2a weißt die Orientierungshilfe R1 einen hinteren Streifen 18 auf, welcher beidens in Seitenstreifen 19 und 20 übergeht. Dabei sind Streifen 18, sowie Seitenstreifen 19, 20 aus elastischen Materialien bspw. aus gepolstertem Goretex mit verbiegbaren Elementen hergestellt. Die Orientierungshilfe R1 kann so einer Schulterform angepaßt werden. Dabei liegt der Streifen 18, am Rücken an, wobei die Seitenstreifen 19 und 20 eine Schulter eines menschlichen Körpers, wie in Figur 3 dargestellt, übergreifen.

Da der elektrische Kompaß 8 in dem Streifen 18 des Gürtels 1 in der Ebene des Rückens festgelegt ist, läßt sich senkrecht zur Rückenebene, also in Gehrichtung nach vorne, diese Richtungskoordinate leicht bestimmen.

Dort ist auch erkennbar, wie die Orientierungshilfe R um den Bauch bzw. Hüfte des menschlichen Körpers befestigt ist. Diese kann allerdings unter der Kleidung unsichtbar getragen werden. Hingegen liegt die Orientierungshilfe R1 bevorzugt auf den Schultern des Blinden auf und hat gleichzeitig eine optische Funktion, da durch Ausgestaltung in leuchtenden oder reflektierenden Farben der Blinde im Dunkeln von Radfahrern, Autos und der Umgebung besser erkannt werden kann.

Damit der Streifen 18 an unterschiedlich breite Schultern angepaßt werden kann, sind flexibel zusammenschiebbare bzw. auseinanderziehbare Brücken 26 in dem Streifen 18 vorgesehen. Um bspw. eine Breite des Streifens 18 zu verringern, können diese Brücken zusammen geschoben werden, wobei auch dran gedacht ist, diese herauszunehmen.

Die Orientierungshilfe R1 weißt im vorderen Bereich des Seitenstreifens 19 einen Signalgeber 21 mit Schalter 22 auf, welcher mit einem bevorzugt runden bis halbkugel- bzw. kugelartigen Sensor 23 verbunden ist und mit dem Signalgeber 21 bzw. 24 über hier nicht näher dargestellte elektrische Verbindungen zu einem in dem Streifen 18 angeordneten Prozessor 7.1 in Verbindung steht. Ein zweiter Signalgeber 24 ist dem Seitenstreifen 20 zugeordnet, welcher ebenfalls mit einem Schalter 25 versehen ist.

Wesentlich ist hier, daß der Sensor 23, welcher als Infrarot, Ultraschall oder sonstiger Sensor ausgebildet sein kann, Signale von Hindernissen, die in einem Bereich etwa 0,5 bis 1,5 m vor dem Sensor 23 auftreten sowohl in Höhe als auch in Breite entsprechend dem Oberkörperbereich von diesem Sensor 23 erkannt werden.

Dieses Signal wird dem Prozessor 7.1 übermittelt, welcher von einem Akku 9.1 gespeist wird, wobei ein Warnsignal von dem Signalgeber 21, 24 in Form von einem Ton, Dauerton od.dgl. abgegeben werden kann.

Der Blinde wird so vor einem Hindernis gerade im Oberkörperbereich gewarnt und kann sich entsprechend darauf einstellen.

Wesentlich aber ist auch, daß die Reichweite des Sensors 23 auf ein Minnimum von ca. 0,5 bis 1,5m begrenzbar ist, da sonst Hindernisse in größerer Entfernung zur Orientierung bzw. zur Warnung den blinden Menschen nur verwirren würden.

Die Orientierungshilfe R1 wird durch den Druckschalter 22 eingeschaltet. Bei einem Hindernis, welches von dem Sensor 23 ermittelt wird, wird optional, geschaltet durch den Schalter 25, ein akustisches Signal in den Signalgebern 21, 24 erzeugt oder aber ein anderes Zeichen bzw. ein anderes Signal bspw. ein mechanisches Vibrieren in Form eines Vibrators. Vibratoren eignen sich besonders als Signalgeber 21, 24, da diese leise sind und trotzdem gut gespürt werden, sodaß Umgebungsgeräusche dadurch nicht beeinträchtigt insbesondere von Blinden überhört werden können.

In einem weiter Ausführungsbeispiel gemäß Figur 2b ist eine Orientierungshilfe R2 gezeigt, welche als identisches Bauteil entsprechend der Orientierungshilfe R1 ausgebildet ist, doch mit dem Unterschied, daß zusätzlich in dem Streifen 18 ein, wie in Figur 1 beschriebener, elektrischer Kompaß 8.1 zugeschaltet ist. Dieser elektrische Kompaß 8.1 übernimmt gleiche Funktionen wie die Orientierungshilfe R aus Figur 1, wobei hier gleichfalls über Betätigen des Schalters 13 des Stabes 10 (Figur 4) ein Einschalten bzw. Speichern der aktuellen Orientierung möglich ist, wobei das Abweichen von dem festgespeicherten Wert in den Signalgebern 21 bzw. 24 je nach Richtungsänderung akustisch oder elektromechanisch zur Anzeige gebracht wird. So kann hier der Blinde zusätzlich, wie in Figur 1 beschrieben, die Orientierungshilfe R2 zusätzlich als Richtungsweiser verwenden.

Im Rahmen der vorliegenden Erfindung soll jedoch auch liegen, daß bspw. ein GPS- System ein Bestandteil der Orientierungshilfe R, R1 oder R2 sein kann. Dort kann dann eine fest vorgegebene einprogrammierte Richtung abgerufen werden, die anschließend über die Signaleinrichtungen 5, 6 bzw. Signalgeber 21, 24 dem Blinden den Weg zu seinem Ziel vermittelt.

## Patentansprüche

1. Orientierungshilfe für Sehbehinderte und blinde Menschen mit einem Speicher (7) zum Abspeichern einer beliebig wählbaren Gehrichtung und wenigstens einer Signaleinrichtung (5, 6) zum Anzeigen einer Änderung von dieser Gehrichtung, mit einem Kompass (8), einem Schalter (13, 22, 25) und einem Prozessor (7,7.1) wobei die wählbare Gehrichtung und somit eine Referenzkoordinate zur Erkennung von Abweichungen von dieser durch die Ausrichtung des Kompasses (8) bei Betätigen des Schalters (13, 22, 25) erfolgt und diese gespeicherte Koordinate als Referenzkoordinate an den Prozessor (7, 7.1) zur Anzeige einer Richtungsänderung an die zumindest eine Signaleinrichtung (5, 6) gelangt, wobei ein aktueller Kompasswert mit der abgespeicherten Referenzkoordinate ständig verglichen wird und eine Änderung zu der abgespeicherten Referenzkoordinate an die zumindest eine Signaleinrichtung (5, 6) weitergibt.

2. Orientierungshilfe nach Anspruch 1, **dadurch gekennzeichnet, daß** zum Erkennen von Koordinaten einer gewählten Gehrichtung ein Kompaß (8) vorgesehen und diesem ein Prozessor (7, 7.1) zum Verarbeiten und Speichern der Koordinaten zugeordnet ist.

3. Orientierungshilfe nach Anspruch 1 oder 2, **dadurch gekennzeichnet, daß** die Signalrichtung (5, 6) einem Gürtel (1) zugeordnet ist.

4. Orientierungshilfe nach Anspruch 3, **dadurch gekennzeichnet, daß** der Gürtel (1) einen vorderen Bereich (2) und einen hinteren Bereich (3) aufweist, wobei die Signaleinrichtungen (5, 6) im vorderen Bereich (2) angeordnet sind.

5. Orientierungshilfe nach Anspruch 4, **dadurch gekennzeichnet, daß** der Gürtel (1) über einen Verschluß (4) verschließbar ist, wobei die Signaleinrichtungen (5, 6) beidseits des Verschlusses (4) verschiebbar auf dem Gürtel (1) angeordnet sind.

6. Orientierungshilfe nach Anspruch 4 oder 5, **dadurch gekennzeichnet, daß** der Prozessor (7), der Kompaß (8) und ein Energieträger (9) in dem hinteren Bereich (3) des Gürtels (1) angeordnet sind.

7. Orientierungshilfe nach wenigstens einem der Ansprüche 1 bis 9, **dadurch gekennzeichnet, daß** einem Streifen (18) beidends Seitenstreifen (19, 20) zugeordnet sind, die über wenigstens eine Schulter eines Benutzers legbar sind, wobei der Streifen (18) die Steitenstreifen (19, 20) miteinander verbindet und die Seitenstreifen (19, 20) eine menschliche Schulter übergreifen.

8. Orientierungshilfe nach Anspruch 10, **dadurch gekennzeichnet, daß** die Seitenstreifen (19, 20) mit Signalgebern (21, 24) versehen sind.

9. Orientierungshilfe nach Anspruch 11, **dadurch gekennzeichnet, daß** dem Signalgeber (21, 24) zumindest ein Sensor (23) zugeordnet ist, welcher mit dem Prozessor (7.1) in Verbindung steht und als Infrarot-, Ultraschall-, Mikrowellen- oder Kurzwellensensor zum Erkennen von Hindernissen im Bereich von 0,5 bis 1,5 m Entfernung ausgebildet ist.

10. Orientierungshilfe nach Anspruch 11 oder 12, **dadurch gekennzeichnet, daß** den Signalgebern (21, 24) Schalter (22, 25) zugeordnet sind.

11. Orientierungshilfe nach wenigstens einem der Ansprüche 10 bis 13, **dadurch gekennzeichnet, daß** dem Streifen (18) der Kompaß (8.1) zugeordnet ist.

## Claims

1. Orientation aid for visually impaired and blind persons comprising of a memory (7) for the storing of a free selected direction of walking, at least one signal device (5,6 ) which indicates a change from such a direction of walking, a compass (8), a switch (13,22,25), and a processor (7, 7.1), at which a selected direction of walking and consequently a reference coordinate for the detection of a deviation from it results from the orientation of the compass (8) when operating the switch (13,22,25) and this stored coordinate becomes a reference coordinate for the processor (7, 7.1) for the indication of a change of direction to at least one signal device (5,6), at which an current coordinate of the compass will be continuously compared with the stored reference coordinate and a deviation to the stored reference coordinate will be transferred to at least one signal device (5,6 ).

2. Orientation aid according to claim 1, **characterized in that** the detection from the coordinates of a selected direction of walking a compass (8) is used which is attached to a processor (7,7.1) for processing and storing of the coordinates.

3. Orientation aid according to claim 1 or 2,**characterized in that** the signal device (5,6 ) is attached to a belt (1).

4. Orientation aid according to claim 3, **characterised in that** the belt (1) has a front area (2) and a rear area (3), at which the signal devices (5,6) are placed in the front area (2).

5. Orientation aid according to claim 4, **characterised in that** the belt (1) can be closed by a fastening device (4), at which the signal devices (5, 6) are adjustable and attached to the belt (1) on both sides of the fastening device (4).

6. Orientation aid according to claim 4 or 5, **characterised in that** the processor (7), the compass (8) and a energy source (9)is placed in the rear area (3) of the belt (1).

7. Orientation aid according to at least one claim of 1 to 6, **characterised in that** a stripe (18) on both sides is attached to side stripes (21,24), which can be placed at least upon one shoulder of the user, at which the stripe (18) joins the side strips (19, 20) and the side strips (19,20) overlap a human shoulder.

8. Orientation aid according to claim 7, **characterised in that** the side strips (19, 20) are supplied with signal devices (21, 24).

9. Orientation aid according to claim 8, **characterised in that** the signal device (21,24) is joined to at least one sensor (23), which is joined to the processor (7.1) and which is designed as infrared-, ultrasonic-, microwave-, or short-wave sensor to detect obstacles in the range of 0.5 m to 1.5 m distance.

10. Orientation aid according to claim 8 or 9, **characterised in that** switches (22, 25) are attached to the signal devices (21, 24).

11. Orientation aid according to at least one claim of 7 to 10, **characterised in that** the compass (8.1) is attached to the strip (18).

## Revendications

1. Aide technique à l'orientation pour malvoyants et personnes aveugles avec une mémoire (7) pour mémoriser une direction de déplacement librement choisie, et au minimum un
dispositif de signalisation (5, 6) pour indiquer une modification de cette direction de déplacement, avec une boussole (8), un interrupteur (13, 22, 25) et un processeur (7, 7.1), en ce que la direction de déplacement choisie et donc une coordonnée de référence pour la perception de déviations de celle-ci se fait par l'alignement de la boussole (8) en actionnant l'interrupteur (13, 22, 25) et que cette coordonnée mémorisée est transmise au processeur (7, 7.1) comme coordonnée de référence pour l'annonce d'un changement de direction au dispositif de signalisation minimum (5, 6), en ce qu'une actuelle donnée de boussole est constamment comparée avec la coordonnée de référence mémorisée et qu'une modification par rapport à la coordonnée de référence mémorisée est transmise au dispositif de signalisation minimum (5, 6).

2. Aide technique à l'orientation selon la revendication 1, **caractérisée en ce qu'**une boussole (8) est prévue pour la perception des coordonnées d'une direction de déplacement choisie, et qu'un processeur (7, 7.1) lui est attribué pour le traitement et la mémorisation des coordonnées.

3. Aide technique à l'orientation selon la revendication 1 ou 2, **caractérisée en ce que** le dispositif de signalisation (5, 6) est attribué à une ceinture (1).

4. Aide technique à l'orientation selon la revendication 3, **caractérisée en ce que** la ceinture présente un secteur avant (2) et un secteur arrière (3), et que les dispositifs de signalisation (5, 6) sont disposés dans le secteur avant (2).

5. Aide technique à l'orientation selon la revendication 4, **caractérisée en ce que** la ceinture (1) est verrouillable par une fermeture (4), et que les dispositifs de signalisation (5, 6) sont disposées de part et d'autre de la fermeture (4) et sont déplaçables sur la ceinture (1).

6. Aide technique à l'orientation selon la revendication 4 ou 5, **caractérisée en ce que** le processeur (7), la boussole (8) et une alimentation (9) sont disposées dans le secteur arrière (3) de la ceinture (1).

7. Aide technique à l'orientation selon au moins une des revendications de 1 à 6, **caractérisée en ce que** des bandes latérales (19, 20) pouvant se passer par-dessus au moins une épaule d'un utilisateur sont attribuées aux deux bouts d'une bande (18), que la bande (18) relie les bandes latérales (19, 20) et que les bandes latérales (19, 20) passent par-dessus l'épaule d'une personne.

8. Aide technique à l'orientation selon la revendication 7, **caractérisée en ce que** des donneurs de signal (21, 24) sont disposés sur les bandes latérales (19, 20).

9. Aide technique à l'orientation selon la revendication 8, **caractérisée en ce qu'**au moins un capteur (23), réalisé par un capteur à infrarouges, à micro-ondes, ou à ondes courtes et servant à la perception d'obstacles dans un rayon de 0,5 à 1,5 m de distance, est connecté au processeur (7.1) et attribué au donneur de signal (21, 24).

10. Aide technique à l'orientation selon la revendication 8 ou 9, **caractérisée en ce que** des interrupteurs (22, 25) sont attribués aux donneurs de signal (21, 24).

11. Aide technique à l'orientation selon au moins une des revendications de 7 à 10, **caractérisée en ce que** la boussole (8.1) est attribuée à la bande (18).
